# EUROPEAN PATENT SPECIFICATION

(11) **EP 2 851 045 B1**
(45) Date of publication and mention of the grant of the patent: **28.09.2016**
(21) Application number: 13791043.6
(22) Date of filing: 25.04.2013
(51) Int. Cl.: A61F 13/15, A61F 13/472, A61F 13/475, A61F 13/534, A61F 13/53, A61F 13/536, A61F 13/551, A61F 13/56

(54) **ABSORPTIVE ARTICLE**
ABSORBIERENDER ARTIKEL
ARTICLE ABSORBANT

(30) Priority: 18.05.2012 JP 2012114845
(43) Date of publication of application: 25.03.2015
(73) Proprietor: Unicharm Corporation, Ehime 799-0111 (JP)
(72) Inventor: KOSAKA, Shoshi, Kanonji-shi Kagawa 769-1602 (JP); NAKAJIMA, Kaiyo, Kanonji-shi Kagawa 769-1602 (JP)
(74) Representative: Peter, Julian
(86) International application number: PCT/JP2013/062268
(87) International publication number: WO 2013/172176

(56) References cited:
- EP-A1- 0 998 889
- EP-A2- 1 043 004
- WO-A1-2011/080860
- WO-A1-2011/092025
- JP-A- H08 502 177
- JP-A- 2009 153 735
- JP-U- H0 521 936

## Description

### Technical Field

The present invention relates to an absorbent article, such as a light incontinence pad, sanitary napkin or panty liner.

### Background Art

In order to avoid reducing the leakproofness of absorbent articles, by twisting of anti-leakage walls during removal from their individual packaging structures, there are known in the prior art absorbent articles individually packaged with an individual packaging structure folded at two or more prescribed locations at sections without expansion force by an elastic member, and positioned so that at least one folded section overlaps with a connected leakproof groove (PTL 1, for example).

### Citation List

### Patent literature

[PTL 1] : Japanese Unexamined Patent Publication No. 2001-37804

Further prior art relevant for this technical field is disclosed in documents EP 0 998 889 A1, WO 2011/080860 A1 and EP 1 043 004 A2.

### SUMMARY OF INVENTION

### Technical Problem

With the absorbent article described in PTL 1, however, the absorbent article twists at the folded. section when the absorbent article is fitted into underwear, and this can reduce the leakproofness of the absorbent article. Also, in order to reduce the size of the individual package, it is sometimes necessary to individually package the absorbent article by folding the section where the anti-leakage wall is provided.

It is an object of the present invention to provide an absorbent article that can minimize twisting of the absorbent article that occurs when the wearer wears the absorbent article.

### Solution to Problem

In order to solve the aforementioned problems, the present invention employs the following construction. Specifically, the present invention is an absorbent article as defined in claim 1.

### Advantageous Effects of Invention

According to the present invention it is possible to provide an absorbent article that can minimize twisting of the absorbent article that occurs when the wearer wears the absorbent article.

### Brief Description of Drawings

Fig. 1 is a partial cutaway plan view of an absorbent article according to one embodiment of the present invention.
Fig. 2 is an exploded cross-sectional schematic diagram showing a cross-section of Fig. 1 along line A-A.
Fig. 3 is a diagram for illustration of an individually packaged absorbent article according to one embodiment of the present invention.
Fig. 4 is a perspective view of a packaged product comprising an individually packaged absorbent article according to one embodiment of the present invention.
Fig. 5 is a drawing illustrating that the absorbent article according to one embodiment of the present invention can be fitted into underwear without twisting, by pulling the wing sections in the widthwise direction.
Fig. 6 is a drawing illustrating that the absorbent article according to one embodiment of the present invention can be fitted into underwear without twisting, by folding the wing sections toward the clothing side.
Fig. 7 is a plan view of a modified example of an absorbent article according to one embodiment of the present invention.

### Description of Embodiments

An absorbent article according to one embodiment of the present invention will now be explained with reference to the accompanying drawings. However, the present invention is not limited to the embodiment of the absorbent article described below.

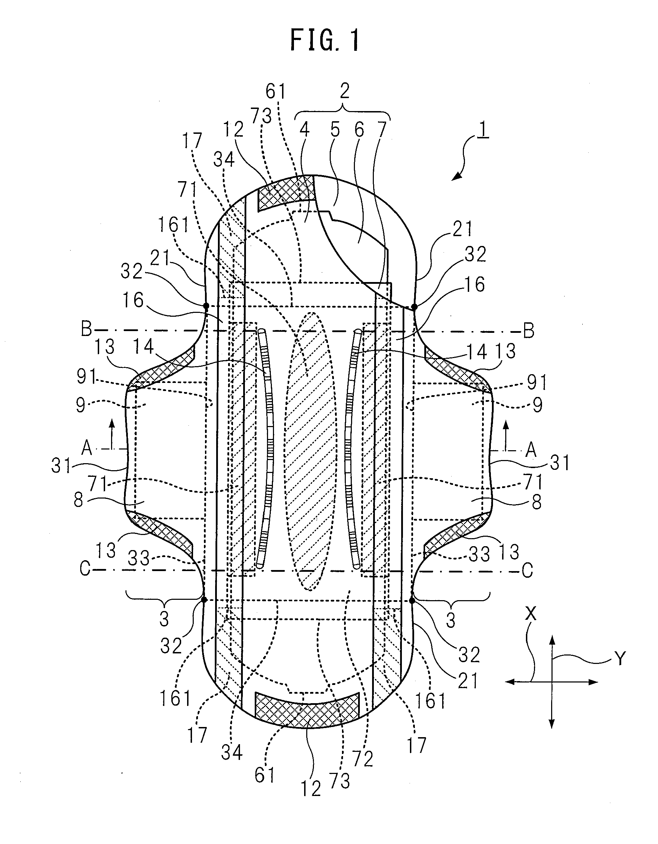

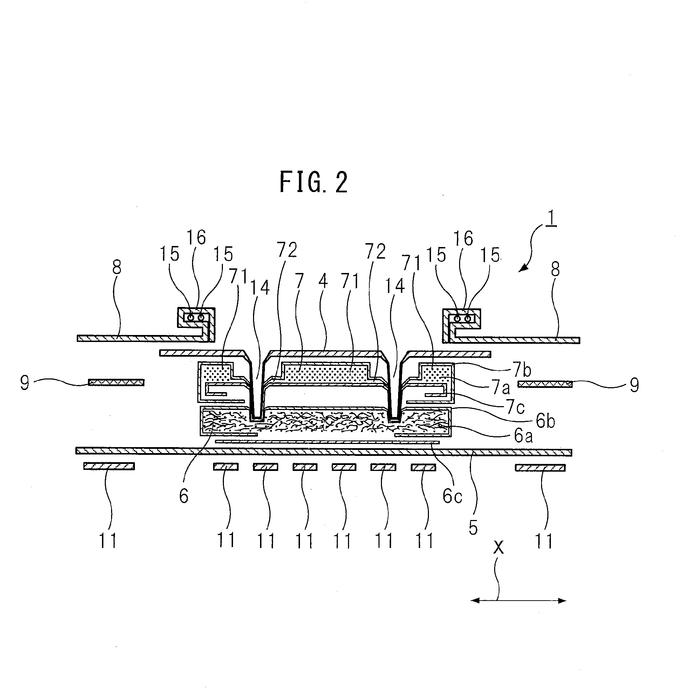

Fig. 1 is a partial cutaway plan view showing an absorbent article according to one embodiment of the invention, and Fig. 2 is an exploded cross-sectional schematic diagram showing a cross-section of Fig. 1 along line A-A. The absorbent article 1 includes a body section 2, and a pair of wing sections 3 extending in the widthwise direction from both edges at the center in the lengthwise direction 21 of the body section 2. The body section 2 comprises a liquid-permeable front sheet 4 provided on the skin side, a liquid-impermeable back sheet 5 provided on the clothing side, a liquid-retaining absorbing layer 6 lying between the front sheet 4 and the back sheet 5, a liquid-retaining super-absorbent polymer sheet 7 lying between the front sheet 4 and the absorbing layer 6, and a pair of side sheets 8 provided on both sides in the widthwise direction of the front sheet 4. Each wing section 3 comprises a back sheet 5 and a side sheet 8, extending in the widthwise direction from the side edge 21 at the center in the lengthwise direction of the body section 2, and a reinforcing sheet 9 lying between the back sheet 5 and the side sheet 8.

A pressure-sensitive adhesive part 11 is preferably provided on the clothing side of the body section 2 and wing sections 3. Also, at the edge section in the lengthwise direction of the body section 2, there is preferably provided a seal section 12 where the front sheet 4 and back sheet 5 are bonded by heat embossing. Furthermore, on both sides in the lengthwise direction of each wing section 3 there is preferably provided a seal section 13 where the side sheet 8 and back sheet 5 are bonded by heat embossing. Also, on the skin side surface of the body section 2 there is preferably provided a pair of compressed grooves 14 extending in the lengthwise direction along the widthwise direction, and reaching from the front sheet 4 to the interior of the absorbing layer 6.

Preferably, the edge sections 16 on the inner side in the widthwise direction of the side sheet 8 are folded over, and a filamentous elastic member 15 is disposed in the folded over sections 16 of the side sheet 8, extending in the lengthwise direction. The folded over sections 16 at the edges on the inner side in the widthwise direction of the side sheet 8 constitutes the anti-leakage walls 16.

In the drawings, the widthwise direction is the X direction and the lengthwise direction is the Y direction.

The shape of the body section 2 is not particularly restricted so long as it is a shape suited to a female body and shape of shorts, such as rectangular, elliptical or hourglass-shaped. The dimensions of extension in the lengthwise direction of the outer shape of the body section 2 are preferably 100 to 500 mm and more preferably 150 to 350 mm. Also, the dimensions of extension in the widthwise direction of the outer shape of the body section 2 are preferably 30 to 200 mm and more preferably 40 to 180 mm.

The front sheet 4 passes body fluids, such as urine or menstrual blood, discharged by the wearer through to the super-absorbent polymer sheet 7 and/or absorbing layer 6 provided under it. All or a part of the front sheet 4 is liquid-permeable, and the liquid-permeable areas of the front sheet 4 may be formed of a resin film with a plurality of liquid-permeable holes formed therein, or a net-like sheet with a plurality of mesh holes, or a liquid-permeable nonwoven fabric or woven fabric. The resin film or net-like sheet may be formed of polypropylene (PP), polyethylene (PE), polyethylene terephthalate (PET) or the like. Also, a nonwoven fabric used for the front sheet 4 may be a spunlace nonwoven fabric formed from cellulose fibers such as rayon or synthetic resin fibers, or an air-through nonwoven fabric formed of synthetic resin fibers, or the like.

The basis weight of the front sheet 4 is preferably 15 to 100 g/m² and more preferably 20 to 50 g/m². If the basis weight of the front sheet 4 is lower than 15 g/m², the front sheet 4 may not have sufficient surface strength or it may potentially tear during use. Also, if the front sheet 4 basis weight is greater than 100 g/m², the front sheet 4 may produce excessive stiffness and discomfort during use. In addition, with prolonged use, a basis weight exceeding 50 g/m² may cause fluids to be held in the front sheet 4, keeping the front sheet 4 continuously in a sticky state and causing discomfort for the wearer.

When the liquid-permeable areas forming all or part of the front sheet 4 is formed by a porous film, such as a film having numerous liquid-permeable holes formed therein, preferably the pore size is in the range of 0.05 to 3 mm, the pitch is in the range of 0.2 to 10 mm, and the pore area ratio is in the range of 3% to 30%.

The back sheet 5 prevents body fluids, such as urine and menstrual blood that have been absorbed into the super-absorbent polymer sheet 7 and/or absorbing layer 6, from leaking to the outside. The material used for the back sheet 5 may be a liquid-impermeable material, such as a resin film. If the material used for the back sheet 5 is one that does not allow passage of body fluids but is air permeable, then it will be possible to reduce mustiness during wear and to reduce discomfort when worn. As examples of such materials there may be mentioned liquid-impermeable films composed mainly of polyethylene (PE) or polypropylene (PP), air-permeable films, and composite sheets obtained by laminating a liquid-impermeable film onto one side of a spunbond or other type of nonwoven fabric. For the back sheet 5 it is preferred to use an impermeable plastic film, or a laminate sheet of a nonwoven fabric and an impermeable plastic film.

The back sheet 5 extends in the widthwise direction near the center in the lengthwise direction, and the section of the back sheet 5 extending in the widthwise direction, together with the section of the side sheet 8 extending in the widthwise direction, forms the wing section 3. Also, the back sheet 5 may be bonded with the front sheet 4, absorbing layer 6 and side sheet 8 using a hot-melt adhesive.

The absorbing layer 6 has the function of absorbing and retaining body fluids, such as urine and menstrual blood. The absorbing layer 6 preferably has high bulk, is resistant to deformation and has low chemical irritation. The absorbing layer 6 used may be, for example, an absorbent body 6a comprising fibers of fluffy pulp, a spunbond nonwoven fabric or airlaid nonwoven fabric and a super-absorbent polymer (SAP: Super Absorbent Polymer), covered with liquid-permeable sheets 6b, 6c, such as tissue. The absorbent body 6a does not need to contain a super-absorbent polymer. For example, a spunbond nonwoven fabric covered with tissue may be used as the absorbing layer.

The absorbent body 6a preferably contains fluffy pulp and a super-absorbent polymer. Instead of fluffy pulp or an airlaid nonwoven fabric, for example, chemical pulp, cellulose fiber or artificial cellulose fiber such as rayon or acetate may be used as the absorbent body fiber. Also, the absorbent body 6a may be composed entirely of fibers, without including a super-absorbent polymer. The basis weight of the fibers, such as pulp, used in the absorbent body 6a is preferably 150 to 700 g/m², and the basis weight of the super-absorbent polymer is preferably 100 to 700 g/m². The mass ratio of the super-absorbent polymer is preferably in the range of 30-100%, where the fibers are 100%.

The basis weight of the liquid-permeable sheets 6b, 6c covering the absorbent body 6a is preferably 12 to 30 g/m². The absorbent body 6a may also be covered with a single liquid-permeable sheet. In addition, the absorbent body 6a may be uncovered with a liquid-permeable sheet, so long as the absorbent body 6a is not deformed.

Examples of airlaid nonwoven fabrics to be used for the absorbent body 6a include nonwoven fabrics comprising pulp and synthetic fiber either heat sealed or fixed with a binder. The super-absorbent polymer to be used in the absorbent body 6a, wherein the water-soluble polymer has a suitably crosslinked three-dimensional network structure, is essentially water-insoluble, although it absorbs a 30- to 60-fold amount of water, and preferably the absorbed water does not emerge even when reasonable pressure is applied. The super-absorbent polymer used may be, for example, a starch-based, acrylic acid-based or amino acid-based particulate or filamentous polymer.

By providing an absorbing layer 6 in the absorbent article 1, it is possible for the absorbent article 1 to absorb body fluids discharged into a super-absorbent polymer-absent region 72 of the super-absorbent polymer sheet 7, which is described below. The absorbent article 1 can also absorb body fluids that cannot be absorbed by the super-absorbent polymer sheet 7, as described below.

A hot-melt adhesive may be coated on the surfaces of the absorbent body 6a sides of the liquid-permeable sheets 6b, 6c. This will anchor the absorbent body 6a to the liquid-permeable sheets 6b, 6c. In addition, the liquid-permeable sheet 6b may be bonded with the liquid-permeable sheet 6c, and the liquid-permeable sheet 6b and liquid-permeable sheet 6c may be integrated and covering the absorbent body 6a.

The super-absorbent polymer (SAP) sheet 7 has the function of absorbing and retaining body fluids, such as urine and menstrual blood, similar to the absorbing layer 6. The super-absorbent polymer sheet 7 includes an absorbent body 7a consisting of a super-absorbent polymer alone, and an upper liquid-permeable sheet 7b and lower liquid-permeable sheet 7c sandwiching the absorbent body 7a composed of a super-absorbent polymer alone. The super-absorbent polymer, similar to the super-absorbent polymer used for the absorbent body 6a, has a suitably crosslinked three-dimensional network structure for the water-soluble polymer, and is essentially water-insoluble although it absorbs a 30- to 60-fold amount of water, and preferably the absorbed water does not emerge even when reasonable pressure is applied. The super-absorbent polymer used may be, for example, a starch-based, acrylic acid-based or amino acid-based particulate or filamentous polymer.

The upper liquid-permeable sheet 7b and lower liquid-permeable sheet 7c used may be a liquid-permeable sheet such as, for example, an air-through nonwoven fabric, a spunbond nonwoven fabric, a hydrophilic treated opening-formed plastic film or a liquid-permeable plastic film. Incidentally, the super-absorbent polymer sheet may also be formed by sandwiching the absorbent body 7a composed of a super-absorbent polymer alone between a single folded liquid-permeable sheet.

The super-absorbent polymer sheet 7 may have a super-absorbent polymer-present region 71 in which the absorbent body 7a composed of a super-absorbent polymer alone is present, super-absorbent polymer-absent region 72 where the absorbent body 7a composed of a super-absorbent polymer alone is essentially absent. In this case, even when the super-absorbent polymer absorbs body fluids and swells, body fluids discharged into the front sheet 4 will not be blocked by the swelled super-absorbent polymer, and will be able to pass through the super-absorbent polymer sheet 7 and migrate into the absorbing layer 6. The phrase "the absorbent body 7a is essentially absent" means not only absence of the absorbent body 7a, but also includes cases where the absorbent body 7a is present, but the presence of the absorbent body 7a is in such a small amount that only very minute amounts of body fluid can be absorbed compared to the amount of absorption of body fluids in the super-absorbent polymer-present region 71.

For example, it is possible to form a super-absorbent polymer-absent region 72 by directly bonding the upper liquid-permeable sheet 7b and the lower liquid-permeable sheet 7c by bonding means using heat embossing, hot-melt adhesive or the like, without sandwiching the absorbent body 7a composed of a super-absorbent polymer alone.

Three super-absorbent polymer-present regions 71, each extending in the lengthwise direction, may be provided along the widthwise direction of the super-absorbent polymer sheet 7. Two of the super-absorbent polymer-present regions 71 among the three super-absorbent polymer-present regions 71 may be provided on either edge side in the widthwise direction of the super-absorbent polymer sheet 7, while the remaining super-absorbent polymer-present region 71 may be provided at the center in the widthwise direction of the super-absorbent polymer sheet 7. Incidentally, it is not necessary to provide a super-absorbent polymer-present region 71 at the center in the widthwise direction of the super-absorbent polymer sheet 7. Furthermore, it is sufficient to provide at least two super-absorbent polymer-present regions 71 in the super-absorbent polymer sheet 7, but four or more super-absorbent polymer-present regions 71 may also be provided in the super-absorbent polymer sheet 7. The basis weight of the absorbent body 7a in the super-absorbent polymer-present region 71 is preferably 100 to 400 g/m².

When body fluids are discharged during use of the absorbent article 1, the absorbent body 7a composed of a super-absorbent polymer alone in the super-absorbent polymer-present region 71 absorbs body fluids and swells. Also, the difference in thickness between the super-absorbent polymer-present region 71 and the super-absorbent polymer-absent region 72 extending in the lengthwise direction is increased in the super-absorbent polymer sheet 7. As a result, the absorbent article 1 tends to bend in the widthwise direction, and thus becomes resistant to bending in the lengthwise direction. Incidentally, the elastic contractive force of the elastic member 15 provided on each anti-leakage wall 16, described hereunder, facilitates bending of the absorbent article 1 in the lengthwise direction. Particularly when the absorbent article 1 is to be used as a light incontinence pad, the elastic contractive force of the elastic member 15 is increased to reliably erect the anti-leakage wall 16 into avoid leakage of large amounts of urine from the absorbent article 1. Consequently, when the absorbent article 1 is to be used as a light incontinence pad, the absorbent article 1 is easily bent in the lengthwise direction by the elastic contractive force of the elastic member 15 provided on the anti-leakage wall 16. Therefore, particularly when the absorbent article 1 is to be used as a light incontinence pad, the effect is further increased by provision of the super-absorbent polymer-present region 71 and super-absorbent polymer-absent region 72 in the super-absorbent polymer sheet 7.

The super-absorbent polymer sheet 7 may be bonded to the front sheet 4 using a hot-melt adhesive. The hot-melt adhesive section (not shown) is coated onto the super-absorbent polymer sheet 7 in a stripe fashion, for example. This is in order to provide the super-absorbent polymer sheet 7 with sections where the hot-melt adhesive is not coated, since body fluid permeability is poor at the sections where the hot-melt adhesive has been coated. Consequently, the method of coating the hot-melt adhesive is not limited to a stripe-coating method, so long as it is a method of coating that allows provision of regions where the hot-melt adhesive is not coated, i.e. a method of coating that allows sections coated with the hot-melt adhesive to be intermittently formed. For example, the hot-melt adhesive may be coated onto the super-absorbent polymer sheet 7 by a non-contact coating method, such as omega coating. The super-absorbent polymer sheet 7 may be further bonded to the absorbing layer 6 using a hot-melt adhesive.

As mentioned above, the super-absorbent polymer sheet 7 is bonded to the front sheet 4, and therefore the crease formed when the absorbent article 1 has been folded over for individual packaging does not easily remain in the absorbent article 1. The absorbent body 7a composed of a super-absorbent polymer alone is resistant to persistence of the crease, compared to an absorbent body containing fibers, such as pulp.

A hot-melt adhesive (not shown) may also be coated onto the surfaces of the absorbent body 7a sides of the upper liquid-permeable sheet 7b and lower liquid-permeable sheet 7c. This will allow the absorbent body 7a to be anchored to the upper liquid-permeable sheet 7b and lower liquid-permeable sheet 7c. It will also allow the upper liquid-permeable sheet 7b to be firmly bonded by the lower liquid-permeable sheet 7c. As shown in Fig. 2, both edge sections where the upper liquid-permeable sheet 7b and lower liquid-permeable sheet 7c are bonded may be folded over inward in the widthwise direction. This will allow the super-absorbent polymer-present regions 71 to be provided near the bases 33 of the wing sections 3.

The pair of side sheets 8 are provided on both sides in the widthwise direction of the front sheet 4. Each side sheet 8 runs along the lengthwise direction, and extends in the widthwise direction near the center of the lengthwise direction. The side sheet 8 preferably has hydrophobicity or water-repellency. A spunbond nonwoven fabric or spunbond/melt blown/spunbond (SMS) nonwoven fabric, for example, may be used for the side sheet 8. In addition, since the side sheet 8 contacts with the skin of the wearer, an air-through nonwoven fabric that can reduce rubbing irritation on the skin is preferably used as the side sheet 8.

As mentioned above, each folded over section 16 at the edge on the inner side in the widthwise direction of the side sheet 8 constitutes an anti-leakage wall 16. The elastic member 15 of the anti-leakage wall 16 is provided on the anti-leakage wall 16 in its extended state in the lengthwise direction (for example, extended to a length of 1.1- to 1.5-fold). Also, both edge sections 17 in the lengthwise direction of the anti-leakage wall 16 are bonded to the front sheet 4 and anchored to the body section 2. Thus, the elastic contractive force of the elastic member 15 functions for bending of the absorbent article 1, and when the wearer has put on the absorbent article 1, the sections where the anti-leakage wall 16 is not bonded to the front sheet 4 (the sections other than both end sections 17 in the lengthwise direction of the anti-leakage wall 16, hereunder referred to as "erect sections of the anti-leakage wall 16") rise up toward the skin side. Also, the anti-leakage wall 16 prevents body fluid from passing through the front sheet 4 and leaking to the outer side of the absorbent article 1. The height of the anti-leakage wall 16, when erect, is preferably 5 to 30 mm.

As mentioned above, the section of the side sheet 8 extending in the widthwise direction, together with the section of the back sheet 5 extending in the widthwise direction, constitutes the wing section 3.

The wing sections 3 are provided in the absorbent article 1 to stably anchor the absorbent article 1 to underwear. After the wing sections 3 have been folded on the outer wall side of the underwear, the wing sections 3 are attached to the crotch region of the underwear through the pressure-sensitive adhesive part 11 to allow the absorbent article 1 to be stably anchored to the underwear. The shapes of the wing sections 3 are essentially rectangular shapes, for example.

Each wing section 3 has a reinforcing sheet 9 that increases the rigidity of the wing section 3. The reinforcing sheet 9 is preferably provided between the side sheet 8 and the back sheet 3. The reinforcing sheet 9 increases the rigidity of the wing section 3 and guides folding of the wing section 3 so that the folded position is near the base 33 of the wing section 3. The sheet to be used for the reinforcing sheet 9 is not particularly restricted so long as it is a sheet that can increase the rigidity of the wing section 3. For example, paper, a nonwoven fabric, a woven fabric, a plastic film or the like may be used as the reinforcing sheet 9. The shape of the reinforcing sheet 9 may be rectangular, for example.

A pressure-sensitive adhesive part 11 is provided on the clothing side of the back sheet 5 of each wing section 3. The pressure-sensitive adhesive part 11 anchors the wing section 3 to the outer side of the crotch region of underwear. A pressure-sensitive adhesive part 11 is provided on the clothing side of the back sheet 5 of the body section 2 as well. The pressure-sensitive adhesive part 11 anchors the body section 2 to the inner side of the crotch region of underwear. The pressure-sensitive adhesive used to form the pressure-sensitive adhesive part 11 may be, for example, one composed mainly of a styrene-based polymer, tackifier or plasticizer.

Styrene-based polymers include styrene-ethylene-butylene-styrene block copolymer, styrene-butylene polymer, styrene-butylene-styrene block copolymer and styrene-isobutylene-styrene copolymer, any of which may be used alone or as polymer blends of two or more. Styrene-ethylene-butylene-styrene block copolymer is preferred as the pressure-sensitive adhesive for the pressure-sensitive adhesive part 11, from the viewpoint of satisfactory thermostability. An organic compound that is solid at ordinary temperature is preferably used as the tackifier and plasticizer. A tackifier may be, for example, a C5 petroleum resin, C9 petroleum resin, dicyclopentadiene-based petroleum resin, rosin-based petroleum resin, polyterpene resin, terpenephenol resin or the like, and a plasticizer may be, for example, a monomer plasticizer such as tricresyl phosphate, dibutyl phthalate or dioctyl phthalate, or a polymer plasticizer, such as a vinyl polymer or polyester.

The pair of compressed grooves 14 provided on the body section 2 halt spreading of body fluid in the widthwise direction, while also allowing efficient migration of body fluid to the absorbing layer 6. Each compressed groove 14 is formed on the body section 2 by compressing the body section 2 in the thickness direction by heat embossing. Preferably, the pair of compressed grooves 14 are each curve inward in the widthwise direction and are symmetrical with respect to the axis running in the lengthwise direction. The spacing of the pair of compressed grooves 14 in the widthwise direction preferably increases from the center in the lengthwise direction toward the outer side in the lengthwise direction. This will allow body fluid discharged near the center in the lengthwise direction of the absorbent article 1 to diffuse in the lengthwise direction, and can minimize leakage of body fluid from the side edges 21 of the body section 2.

The compressed grooves 14 are preferably provided within the region where the super-absorbent polymer sheet 7 is disposed, and more preferably they are provided within the super-absorbent polymer-absent region 72 of the super-absorbent polymer sheet 7. This can prevent tearing of the upper liquid-permeable sheet 7b and/or lower liquid-permeable sheet 7c by the concavoconvexities of the super-absorbent polymer when the compressed grooves 14 are formed by heat embossing. It will also allow the shapes of the compressed grooves 14 to be maintained even when the absorbent body 7a of the super-absorbent polymer sheet 7 has absorbed body fluid and swelled.

The seal sections 12 provided on the end sections in the lengthwise direction of the body section 2 prevent detachment of the front sheet 4 from the back sheet 5 at the end sections in the lengthwise direction of the body section 2. In addition, the seal sections 12 increase rigidity at the edge sections in the lengthwise direction of the body section 2, and minimize rounding of the edge sections in the lengthwise direction of the body section 2.

The seal sections 13 provided on both sides in the lengthwise direction of each wing section 3 prevent detachment of the side sheet 8 from the back sheet 5 at the wing section 3. In addition, the seal sections 13 increase the rigidity of the wing section 3 and facilitate folding of the wing section 3.

The constituent elements of the absorbent article 1 have been explained above, and the positional relationships of each of the constituent elements will now be explained. The criteria for the positions in explaining the positional relationships between the constituent elements are the positions of the edges 32 in the lengthwise direction of the wing sections 3, which are the points where the borders 31 of the wing sections 3 intersect with the side edges 21 of the body section 2, and the folding positions when the absorbent article 1 is folded into three sections in the lengthwise direction for individual packaging of the absorbent article 1 (line B-B and line C-C).

The edges 32 in the lengthwise direction of the wing sections 3 are, for example, the following portions. In the case of an absorbent article where the shape of the body section is rectangular, the edges in the widthwise direction at the sections where the width of the absorbent article, which was constant proceeding in the lengthwise direction from the edges in the lengthwise direction of the absorbent article to the wing sections, has begun to increase, correspond to the edges in the lengthwise direction of the wing sections. Also, in the case of an absorbent article where the shape of the body section is elliptical, the edges in the widthwise direction at the sections where the width of the absorbent article, which was increasing in the lengthwise direction from the edges in the lengthwise direction of the absorbent article to the wing sections, has begun to further drastically increase, correspond to the edges in the lengthwise direction of the wing sections. Also, in the case of an absorbent article wherein the shape of the body section is hourglass-shaped, the edges in the widthwise direction at the sections where the width of the absorbent article, which had been increasing in the lengthwise direction from the edges in the lengthwise direction of the absorbent article to the wing sections, has decreased and then begun increasing again, i.e. the edges in the widthwise direction at the sections where the width of the absorbent article is minimum after the width of the absorbent article, which had been increasing in the lengthwise direction from the edges in the lengthwise direction of the absorbent article to the wing sections, has decreased, correspond to the edges in the lengthwise direction of the wing sections.

The line connecting both edges 32 in the lengthwise direction of the wing section 3 corresponds to the base 33 of the wing section 3, which is the border between the wing section 3 and the body section 2. The line 34 connecting the edges 32 in the lengthwise direction aligned in the widthwise direction of the pair of wing sections 3 will hereunder be referred to as the wing section action line 34.

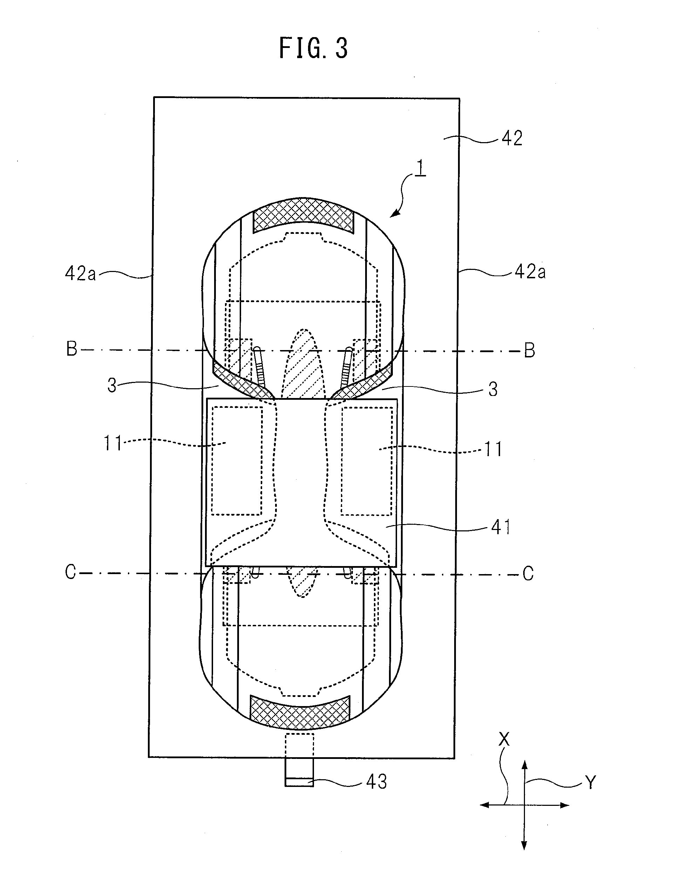

The folding position when the absorbent article 1 has been folded into three sections in the lengthwise direction for individual packaging of the absorbent article 1 (line B-B and line C-C) will now be explained. Fig. 3 is a diagram for illustration of an individually packaged absorbent article. When the absorbent article 1 is to be packaged, the pair of wing sections 3 are folded inward in the widthwise direction, as shown in Fig. 3.

The pressure-sensitive adhesive part 11 is covered by a release sheet 41. This can protect the pressure-sensitive adhesive part 11 before use of the absorbent article 1. The release sheet 41 is not particularly restricted so long as it is a sheet that is releasable from the pressure-sensitive adhesive of the pressure-sensitive adhesive part 11. For example, a substrate coated with a release agent may be used as the release sheet 41. The substrate coated with release agents includes film of polypropylene, low-density polyethylene, polyvinyl alcohol and the like, nonwoven fabrics or paper, and release agents include silicone-based, fluorine-based or isocyanate agents.

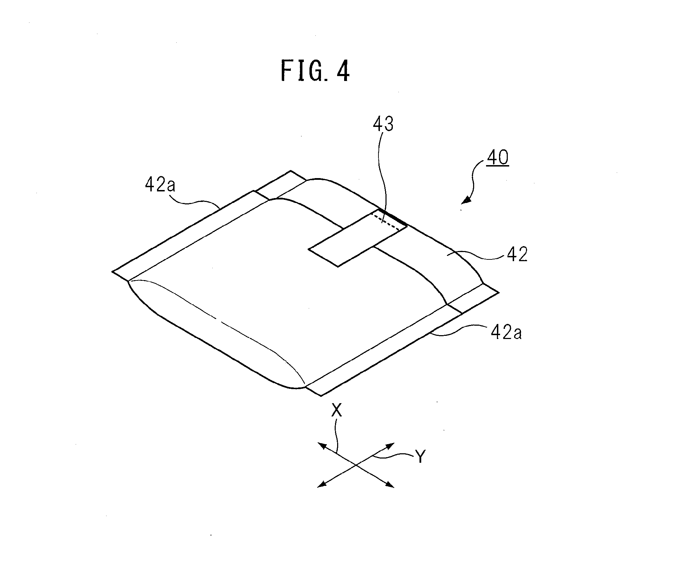

When the absorbent article 1 is packaged, as shown in Fig. 3, the absorbent article 1 with the wing sections 3 folded is placed on a packaging material 42. The packaging material 42 is made from a resin film, nonwoven fabric or the like. Fastening tape 43 is provided on one edge of the packaging material 42 in the lengthwise direction. The absorbent article 1 placed on the packaging material 42 is folded in the lengthwise direction (Y direction) along line B-B and line C-C, together with the packaging material 42. The fastening tape 43 is used to maintain the folded state of the absorbent article 1. Finally, both sides 42a in the widthwise direction of the packaging material 42 folded together with the absorbent article 1 are bonded by heat embossing. This forms a packaged product 40 as shown in Fig. 4, comprising an absorbent article 1. Fig. 4 is a perspective view of a packaged product 40 in which the absorbent article 1 is individually packaged.

Each of the constituent elements of the absorbent article 1 are preferably in the following positional relationships.

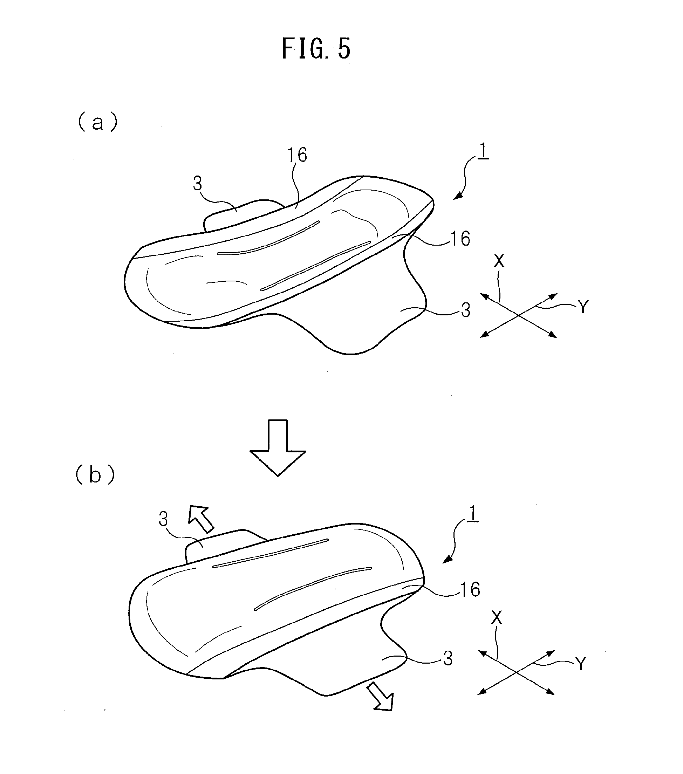

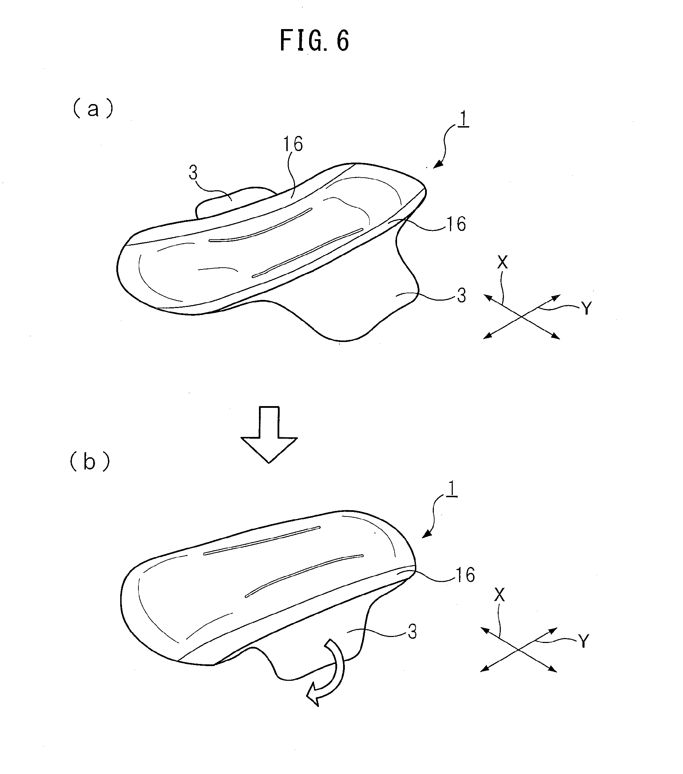

(1) The edges 73 in the lengthwise direction of the super-absorbent polymer sheet 7 are preferably positioned on the outer sides in the lengthwise direction with respect to the wing section action lines 34 connecting the edges 32 in the lengthwise direction that are aligned in the widthwise direction of the pair of wing sections 3. For example, the edges 73 in the lengthwise direction of the super-absorbent polymer sheet 7 may be positioned 10 to 30 mm on the outer side in the lengthwise direction with respect to the wing section action lines 34.
   As shown in Fig. 5(a), the absorbent article 1 bends by elastic contractive force of the elastic member provided on the anti-leakage wall 16, so that the clothing side becomes convex. When this occurs, the absorbent article 1 may become attached to the crotch region of underwear with the absorbent article 1 in a twisted state, without the absorbent article 1 being firmly bonded to the crotch region of the underwear. However, if the pair of wing sections 3 are pulled outward in the widthwise direction, as shown in Fig. 5(b), the absorbent article 1 that was bent becomes flat, and the absorbent article 1 can be attached to the crotch region of underwear with the absorbent article 1 in a non-twisted state. This makes it possible to minimize leakage of body fluids that results when the absorbent article 1 becomes attached to the crotch region of underwear in a twisted state.
   Because the super-absorbent polymer sheet 7 increases the rigidity of the absorbent article 1, positioning the edges 73 in the lengthwise direction of the super-absorbent polymer sheet 7 on the outer side in the lengthwise direction with respect to the wing section action lines 34 connecting the edges 32 in the lengthwise direction of the pair of wing sections 3, as described above, allows the absorbent article 1 that was bent to become flat up to the edge sections in the lengthwise direction of the absorbent article 1 when the pair of wing sections 3 have been pulled outward in the widthwise direction. This allows the absorbent article 1 to be attached to the crotch region of underwear without twisting up to the edge section in the lengthwise direction of the absorbent article 1.
   Since the front sheet 4 and the super-absorbent polymer sheet 7 are bonded as mentioned above, the crease formed when the absorbent article has been folded over for individual packaging does not easily remain. Thus, since the crease formed by folding for individual packaging does not easily remain, the absorbent article 1 can be further flattened up to the edge sections in the lengthwise direction of the absorbent article 1 when the pair of wing sections 3 have been pulled outward in the widthwise direction.
(2) The edges 73 in the lengthwise direction of the super-absorbent polymer sheet 7 are preferably disposed on the outer side in the lengthwise direction, with respect to the folding positions (line B-B and line C-C) when the absorbent article 1 is folded into three sections in the lengthwise direction. As a result, the folding positions (line B-B and line C-C) when the absorbent article 1 has been folded into three sections in the lengthwise direction become positioned within the region where the super-absorbent polymer sheet 7 is provided. As mentioned above, the super-absorbent polymer sheet 7 is bonded to the front sheet 4, and the super-absorbent polymer sheet 7 does not easily retain the creases compared to the absorbent body comprising fiber, such as pulp, and it is therefore the positioning described above makes it possible to minimize residue of the creases formed by folding for individual packaging after the absorbent article 1 has been removed from the packaged product.
(3) The wing section action lines 34 are preferably located at the same positions or at positions on the outer side in the lengthwise direction, with respect to the folding positions (line B-B and line C-C) when the absorbent article 1 is folded into three sections in the lengthwise direction. For example, the wing section action lines 34 may be positioned 0 to 15 mm on the outer side in the lengthwise direction with respect to the folding positions (line B-B and line C-C).
   As shown in Fig. 6(a), the absorbent article 1 bends by elastic contractive force of the elastic member provided on the anti-leakage wall 16, so that the clothing side becomes convex. When this occurs, the absorbent article 1 may become attached to the crotch region of underwear with the absorbent article 1 in a twisted state. However, if the wing section action lines 34 are located at the same positions or at positions on the outer sides in the lengthwise direction, with respect to the folding positions (line B-B and line C-C) when the absorbent article 1 is folded into three sections in the lengthwise direction, when the wing sections 3 are folded toward the clothing side as shown in Fig. 6(b), the absorbent article 1 that was bent becomes flat, and it is possible for the absorbent article 1 to be attached to the crotch region of underwear with the absorbent article 1 in a non-twisted state. Furthermore, this makes it possible to minimize leakage of body fluids that results when the absorbent article 1 becomes attached to the crotch region of underwear in a twisted state.
   Since the front sheet 4 and the super-absorbent polymer sheet 7 are bonded as mentioned above, the crease formed when the absorbent article has been folded over for packaging does not easily remain. Thus, since the crease formed by folding for packaging does not easily remain, the absorbent article 1 can be further flattened when the wing sections 3 are folded toward the clothing side.
(4) The pair of compressed grooves 14 are preferably symmetrical with respect to the axis running in the lengthwise direction, and the spacing of the pair of compressed grooves 14 in the widthwise direction preferably increases from the center in the lengthwise direction toward the outer sides in the lengthwise direction. Since the rigidity is increased at the sections where the compressed grooves 14 are provided, it is possible to prevent twisting of the absorbent article 1 not only in the lengthwise direction of the absorbent article 1 but also across the widthwise direction of the absorbent article 1, when the pair of wing sections 3 have been pulled outward in the widthwise direction as shown in Fig. 5(b), or when the wing sections 3 have been folded toward the clothing side as shown in Fig. 6(b). The spacing of the pair of compressed grooves 14 in the widthwise direction may be maximized at the position where is maximally adjacent to the wing section action lines 34, or at the wing section action lines 34.
(5) The edges of the compressed grooves 14 in the lengthwise direction are preferably positioned on the inner side in the lengthwise direction with respect to the edges 73 in the lengthwise direction of the super-absorbent polymer sheet 7. This will help prevent body fluid discharged near the center in the lengthwise direction of the absorbent article 1 from diffusing in the lengthwise direction beyond the region where the super-absorbent polymer sheet 7 is provided, to help prevent leakage of body fluid from the edges in the lengthwise direction of the body section 2.
(6) The wing section action lines 34 are preferably positioned on the outer side in the lengthwise direction with respect to the edges in the lengthwise direction of the super-absorbent polymer-present region 71 of the super-absorbent polymer sheet 7. If the super-absorbent polymer of the super-absorbent polymer-present region 71 absorbs body fluid and swells, the super-absorbent polymer sheet 7 may undergo twisting. When the super-absorbent polymer sheet 7 twists, body fluid can potentially leak from the absorbent article 1. However, by positioning the wing section action lines 34 on the outer sides in the lengthwise direction with respect to the edges in the lengthwise direction of the super-absorbent polymer-present region 71 of the super-absorbent polymer sheet 7, the super-absorbent polymer-present region 71 as a whole becomes pulled outward in the widthwise direction by the wing sections 3, so that it is possible to minimize twisting of the super-absorbent polymer sheet 7 due to swelling of the super-absorbent polymer of the super-absorbent polymer-present region 71.
(7) The two super-absorbent polymer-present regions 71 are preferably each provided on the outer sides in the widthwise direction of the pair of compressed grooves 14. Since the super-absorbent polymer-present region 71 in the super-absorbent polymer sheet 7 is a particularly high rigidity region, the wing sections 3 can be easily folded near the bases 33 of the wing sections 3.
(8) The edges 61 in the lengthwise direction of the absorbing layer 6 are preferably at the same position or positioned on the outer sides in the lengthwise direction, with respect to the edges 73 in the lengthwise direction of the super-absorbent polymer sheet. This can minimize leakage of body fluid discharged into the absorbent article 1 from the edges in the lengthwise direction of the absorbent article 1.
(9) The edges 161 in the lengthwise direction of the erect sections of the anti-leakage walls 16, which are the sections of the anti-leakage walls 16 that rise up toward the skin side, are preferably positioned on the inner side in the lengthwise direction, with respect to the edges 73 in the lengthwise direction of the super-absorbent polymer sheet 7. This can minimize twisting of the erect sections of the anti-leakage walls 16 when the wing sections 3 have been folded and the absorbent article has been fitted in underwear. In addition, the edges 161 of the erect sections of the anti-leakage walls 16 in the lengthwise direction are preferably positioned on the outer sides in the lengthwise direction with respect to the wing section action lines 34. The sections 17 of the anti-leakage walls 16 bonded to the front sheet 4 at both edges in the lengthwise direction are the sections of increased rigidity. By positioning the edges 161 of the erect sections of the anti-leakage walls 16 in the lengthwise direction on the outer sides in the lengthwise direction with respect to the wing section action lines 34, it is possible to fold the wing sections 3 without them being affected by these sections with increased rigidity, thus allowing the wing sections 3 to be easily folded near the bases 33.
(10) The edges 161 of the erect sections of the anti-leakage walls 16 in the lengthwise direction, which are the sections of the anti-leakage walls 16 that rise up toward the skin side, are preferably positioned on the outer sides in the lengthwise direction with respect to the wing section action lines 34. This will allow the anti-leakage walls 16 to more reliably prevent leakage of body fluids to the outside of the absorbent article 1.
(11) The edges 91 on the inner side in the widthwise direction of the reinforcing sheet 9 are preferably at the same positions or are positioned on the outer side in the widthwise direction, with respect to the bases 33 of the wing sections 3, and more preferably they are at the same positions with respect to the bases 33 of the wing sections 3. This will allow the wing sections 3 to be folded near the bases 33 of the wing sections 3 when the absorbent article 1 is attached on underwear.
(12) The edges on the inner side in the widthwise direction of the seal sections 13 provided on both sides in the lengthwise direction of the wing sections 3, are preferably at the same positions or positioned on the outer sides in the widthwise direction, with respect to the bases 33 of the wing sections 3, and more preferably they are at the same positions with respect to the bases 33 of the wing sections 3. Because the sections of the wing sections 3 where the seal sections 13 are provided have high rigidity, the wing sections 3 can be folded near the bases 33 of the wing sections 3.

The absorbent article 1 according to one embodiment of the present invention may also be modified as follows. The same constituent elements as the absorbent article 1 according to the aforementioned embodiment of the present invention will be referred to by the same reference numerals, and the explanation will focus on the elements differing from the absorbent article 1 according to the aforementioned embodiment of the present invention.

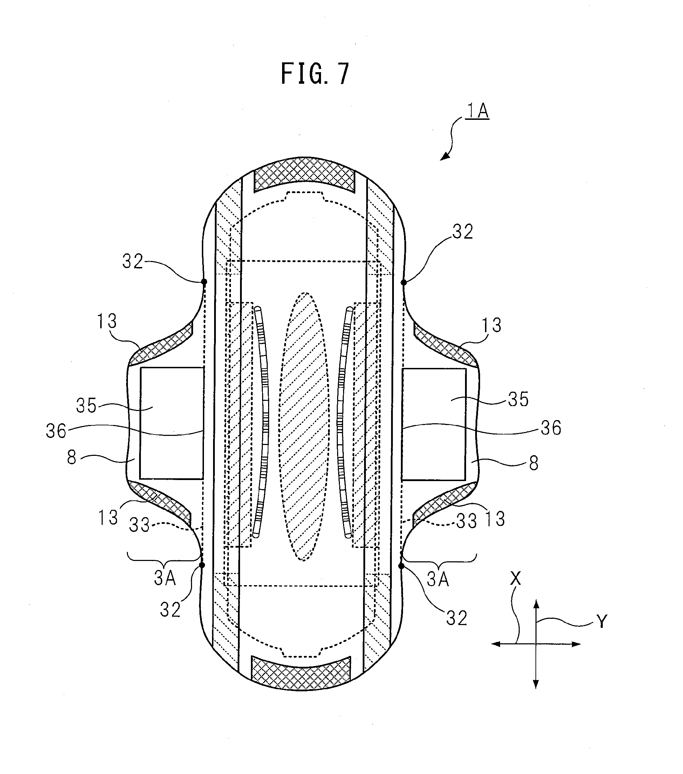

(1) Openings 35 running through the thickness direction may be provided in the wing sections 3A, as in the absorbent article 1A illustrated in Fig. 7. In this case, the edges 36 of the openings 35 on the inner side in the widthwise direction are preferably at the same positions or positioned on the outer sides in the widthwise direction, with respect to the bases 33 of the wing sections 3A, and more preferably they are at the same positions with respect to the bases 33 of the wing sections 3A. Since the rigidity of the wing sections 3A is lower near the bases 33 of the wing sections 3A, the wing sections 3A can be easily folded near the bases 33 of the wing sections 3A when the absorbent article 1A is fitted in underwear. Also, seal sections 13 are preferably provided on both sides in the lengthwise direction of the wing sections 3A. This can increase the tensile strength in the widthwise direction of the wing sections 3A that has been reduced due to provision of the openings 35.
(2) In the embodiment described above, wing sections 3 were formed with the side sheet 8 forming the anti-leakage walls 16 at the edges on the inner side in the widthwise direction and the back sheet 5, but alternatively the wing sections may be formed using separate members from the side sheet and back sheet. For example, separate members from the side sheet and back sheet may be provided on both sides in the widthwise direction of the body section to form the wing sections. Also, separate members from the side sheet forming the anti-leakage walls at the edges on the inner side in the widthwise direction may be provided on the outer sides in the widthwise direction of the side sheet, to form wing sections by the separate members from the side sheet, and the back sheet.

The aforementioned embodiment may also be applied in combination with modifications.

The explanation provided above is merely for illustration, and the present invention is in no way restricted by the described embodiments.

### Industrial Applicability

The absorbent article of the invention may be used as an incontinence pad, sanitary napkin, panty liner or the like, but it is particularly suitable for use as a light incontinence pad. A light incontinence pad is a product that is usually used for longer periods than a sanitary napkin and must therefore have a construction designed for long-term use, and since the absorbent article of the present invention can be anchored to underwear in a stable manner for prolonged periods, it is particularly suitable for use in a light incontinence pad.

### Reference Sign List

- 1, 1A: Absorbent articles
- 2: Body section
- 3: Wing section
- 4: Front sheet
- 5: Back sheet
- 6: Absorbing layer
- 6a: Absorbent body
- 6b, 6c: Liquid-permeable sheets
- 7: Super-absorbent polymer sheet
- 7a: Absorbent body
- 7b: Upper liquid-permeable sheet
- 7c: Lower liquid-permeable sheet
- 8: Side sheet
- 9: Reinforcing sheet
- 11: Pressure-sensitive adhesive part
- 12, 13: Seal sections
- 14: Compressed groove
- 15: Elastic member
- 16: Anti-leakage wall
- 21: Side edge of body section
- 31: Border of wing section
- 32: Edge in lengthwise direction of wing section
- 33: Base of wing
- 34: Wing section action line
- 35: Opening
- 36: Edge on inner side in widthwise direction of opening
- 40: Packaged product
- 41: Release sheet
- 42: Packaging material
- 61: Edge in lengthwise direction of absorbing layer
- 71: Super-absorbent polymer-present region
- 72: Super-absorbent polymer-absent region
- 73: Edge in lengthwise direction of super-absorbent polymer sheet
- 91: Edge on inner side in widthwise direction of reinforcing sheet
- 161: Edge in lengthwise direction of erect section of anti-leakage wall

## Claims

1. An absorbent article having a lengthwise direction and a widthwise direction, and including a body section (2) comprising a liquid-permeable front sheet (4) provided on a skin side, a liquid-impermeable back sheet (5) provided on a clothing side, and a liquid-retaining absorbing layer (6) provided between the front sheet (4) and the back sheet (5), and having a liquid-retaining super-absorbent polymer sheet (7) provided between the front sheet (4) and the absorbing layer (6) and including an absorbent body composed only of a super-absorbent polymer, with liquid-permeable sheets (6b, 6c) sandwiching the absorbent body, and a pair of wing sections (3) extending in the widthwise direction from both edges (21) at the center in the lengthwise direction of the body section (2),
wherein the front sheet (4) and the super-absorbent polymer sheet (7) are bonded,
the pair of wing sections (3) each have an edge (32) in the lengthwise direction,
the positions of the edges (32) in the lengthwise direction of the wing sections (3) each is the point where the borders of the wing sections (3) intersect with the side edges (21) of the body section (2),
wing section action lines (34) are defined along the widthwise direction by connecting the edges (32) of said wing sections (3),
edges (73) in the lengthwise direction of the super-absorbent polymer sheet (7) are positioned on the outer side in the lengthwise direction, with respect to the wing section action lines (34), and
the wing section action lines (34) are positioned at the same positions or on the outer sides in the lengthwise direction, with respect to a folding position when the absorbent article (1, 1A) is folded into three sections in the lengthwise direction for individual packaging of the absorbent article (1, 1A).

2. The absorbent article according to claim 1, wherein:
the super-absorbent polymer sheet (7) has two or more super-absorbent polymer-present regions (71) where the absorbent body is present, and a super-absorbent polymer-absent region (72) where the absorbent body is essentially absent,
the two or more super-absorbent polymer-present regions (71) extending in the lengthwise direction and being aligned in the widthwise direction, and
the wing section action lines (34) being positioned on the outer sides in the lengthwise direction with respect to the edges in the lengthwise direction of the two or more super-absorbent polymer-present regions (71).

3. The absorbent article according to claim 1 or 2, wherein:
a pair of compressed grooves (14), formed by compression of the body section (2) in the thickness direction by heat embossing, extending in the lengthwise direction and aligned in the widthwise direction, and reaching from the front sheet (4) to the interior of the absorbing layer, are provided on the skin side of the body section (2),
the spacing of the pair of compressed grooves (14) in the widthwise direction increasing from the center in the lengthwise direction toward the outer sides in the lengthwise direction.

4. The absorbent article according to claim 3, wherein the edges of the compressed grooves (14) in the lengthwise direction are positioned on the inner sides in the lengthwise direction with respect to the edges in the lengthwise direction of the super-absorbent polymer sheet (7).

5. The absorbent article according to claim 3 or 4, wherein two of the super-absorbent polymer-present regions (71) of the two or more super-absorbent polymer-present regions (71) are provided on the respective outer sides of the pair of compressed grooves (14) in the widthwise direction.

6. The absorbent article according to any one of claims 1 to 5, wherein the edges in the lengthwise direction of the absorbing layer (6) are at the same positions or positioned on the outer sides in the lengthwise direction, with respect to the edges in the lengthwise direction of the super-absorbent polymer sheet (7).

7. The absorbent article according to any one of claims 1 to 6, wherein:
the body section (2) further comprises a pair of side sheets (8) provided on both sides in the widthwise direction of the front sheet (4),
the side sheets (8) have anti-leakage walls (16) on the edge sections on the inner side in the widthwise direction, and
the edges in the lengthwise direction of the erect sections, that rise up toward the skin side of the anti-leakage walls (16), are positioned on the inner sides in the lengthwise direction, with respect to the edges in the lengthwise direction of the super-absorbent polymer sheet (7).

8. The absorbent article according to any one of claims 1 to 6, wherein:
the body section (2) further comprises a pair of side sheets (8) provided on both sides in the widthwise direction of the front sheet (4),
the side sheets have anti-leakage walls (16) on the edge sections on the inner side in the widthwise direction, and
the edges in the lengthwise direction of the erect sections, that rise up toward the skin side of the anti-leakage walls (16), are positioned on the outer sides in the lengthwise direction, with respect to the wing action lines (34).

9. The absorbent article according to any one of claims 1 to 8, wherein:
the wing sections (3) each have a reinforcing sheet (9) that increases the rigidity of the wing section (3), and
the edges on the inner sides in the widthwise direction of the reinforcing sheet (9) are positioned at the same positions or are positioned on the outer sides in the widthwise direction, with respect to lines connecting both edges of the wing sections (3) in the lengthwise direction.

10. The absorbent article according to any one of claims 1 to 6, wherein:
the body section (2) further comprises a pair of side sheets (8) provided on both sides in the widthwise direction of the front sheet (4),
the wing sections (3) each comprise the back sheet (5) and the side sheet (8), extending in the widthwise direction from the side edge at the center in the lengthwise direction of the body section (2),
the wing sections (3) each have a seal section (13) formed by bonding the side sheet (8) and back sheet (5) by heat embossing, on both sides in the lengthwise direction, and
the edges on the inner side of the seal section (13) in the widthwise direction are positioned at the same positions or are positioned on the outer sides in the widthwise direction, with respect to lines connecting both edges of the wing sections (3) in the lengthwise direction.

11. The absorbent article according to any one of claims 1 to 8, wherein:
the wing sections (3) each have an opening (35) running through the thickness direction, and
the edges on the inner sides in the widthwise direction of the openings (35) are positioned at the same position or are positioned on the outer sides in the widthwise direction, with respect to lines connecting both edges of the wing sections (3) in the lengthwise direction.

## Patentansprüche

1. Saugfähiger Artikel mit einer Längsrichtung und einer Breitenrichtung und mit einem Körperabschnitt (2), der eine flüssigkeitsdurchlässigen Vorderlage (4) vorgesehen auf einer Hautseite, eine flüssigkeitsundurchlässige Rücklage (5) vorgesehen auf einer Kleidungsseite, und eine flüssigkeitsspeichernde Absorptionslage (6) vorgesehen zwischen der Vorderlage (4) und der Rücklage (5), umfasst und eine flüssigkeitsspeichernde Superabsorber-Polymerlage (7) vorgesehen zwischen der Vorderlage (4) und der Absorptionslage (6) aufweist und mit einem Absorptionskörper bestehend aus nur einem Superabsorber-Polymer, mit flüssigkeitsdurchlässigen Lagen (6b, 6c) zwischen denen der Absorptionskörper liegt, und einem Paar von Flügelabschnitten (3), die sich in die Breitenrichtung, von beiden Kanten (21) an der, in Längsrichtung, Mitte des Körperabschnitts (2), erstrecken,
wobei die Vorderlage (4) und die Superabsorber-Polymerlage (7) verklebt sind,
das Paar von Flügelabschnitten (3) jeweils eine Kante (32) in der Längsrichtung aufweist,
die Position der Kanten (32) in der Längsrichtung der Flügelabschnitte (3) jeweils der Punkt ist, bei dem die Grenzen der Flügelabschnitte (3) mit den Seitenkanten (21) des Körperabschnitts (2) kreuzen,
Flügelabschnitt-Aktionslinien (34) entlang der Breitenrichtung durch Verbinden der Kanten (32) des Flügelabschnitts (3) bestimmt sind,
Kanten (73) in der Längsrichtung der Superabsorber-Polymerlage (7) in der Längsrichtung auf der Außenweite, bezogen auf die Flügelabschnitt-Aktionslinien (34), angeordnet sind, und
die Flügelabschnitt-Aktionslinien (34) an derselben Position oder in der Längsrichtung auf den Außenseiten, bezogen auf eine Falteposition wenn der saugfähige Artikel (1, 1A) in Längsrichtung in drei Abschnitte für die Einzelverpackung des saugfähigen Artikels (1, 1A) gefaltet wird, angeordnet sind.

2. Saugfähiger Artikel nach Anspruch 1, wobei:
die Superabsorber-Polymerlage (7) zwei oder mehr superabsorberpolymerhaltige Bereiche (71) aufweist in denen der Absorptionskörper vorhanden ist, und einen superabsorber-polymerlosen Bereich (72) in dem der Absorptionskörper im Wesentlichen nicht vorhanden ist,
die beiden oder mehr superabsorber-polymerhaltigen Bereiche (71) sich in die Längsrichtung erstrecken und in die Breitenrichtung ausgerichtet sind, und
die Flügelabschnitt-Aktionslinien (34) auf den Außenseiten, in der Längsrichtung, bezogen auf die Kanten, in der Längsrichtung, der zwei oder mehr superabsorber-polymerhaltigen Bereiche (71) angeordnet sind.

3. Saugfähiger Artikel nach Anspruch 1 oder 2, wobei:
ein Paar eingedrückte Nuten (14), gebildet durch Kompression des Körperabschnitts (2) in Dickenrichtung durch Heißprägen, sich in die Längsrichtung erstreckend und in die Breitenrichtung ausgerichtet und von der Vorderlage (4) in das Innere der Absorptionslage reichend, auf der Hautseite des Körperabschnitts (2) vorgesehen sind,
der Abstand des Paars eingedrückter Nuten (14) in der Breitenrichtung von der Mitte in Längsrichtung in Richtung der Außenseiten in Längsrichtung zunimmt.

4. Saugfähiger Artikel nach Anspruch 3, wobei die Kanten in die Längsrichtung der eingedrückten Nuten (14) in der Längsrichtung auf der Innenseite in der Längsrichtung bezogen auf die Kanten in der Längsrichtung der Superabsorber-Polymerlage (7) angeordnet sind.

5. Saugfähige Artikel nach Anspruch 3 oder 4, wobei zwei der superabsorberpolymerhaltigen Bereiche (71) der zwei oder mehr superabsorberpolymerhaltigen Bereiche (71) auf den jeweiligen Außenseiten in der Breitenrichtung des Paars der eingedrückten Nuten (14) vorgesehen sind.

6. Saugfähiger Artikel nach einem der Ansprüche 1 bis 5, wobei die Kanten in der Längsrichtung der Absorptionslage (6) an denselben Positionen oder auf den Außenseiten in der Längsrichtung bezogen auf die Kanten in der Längsrichtung der Superabsorber-Polymerlage (7) angeordnet sind.

7. Saugfähiger Artikel nach einem der Ansprüche 1 bis 6, wobei:
der Körperabschnitt (2) ferner ein Paar von Seitenlagen (8), vorgesehen auf beiden Seiten in der Breitenrichtung der Vorderlage (4), umfasst,
die Seitenlagen (8) Antileckagewände (16) auf dem Kantenabschnitt auf der Innenseite in die Breitenrichtung umfassen, und
die Kanten in die Längsrichtung der Aufrichtabschnitte, die sich in Richtung der Hautseite der Antileckagewände (16) erheben, auf der Innenseite in die Längsrichtung, bezogen auf die Kanten in die Längsrichtung der Superabsorber-Polymerlage (7), angeordnet sind.

8. Saugfähiger Artikel nach einem der Ansprüche 1 bis 6, wobei:
der Körperabschnitt (2) ferner ein Paar Seitenlagen (8), vorgesehen auf beiden Seiten in der Breitenrichtung der Vorderlage (4), umfasst,
die Seitenlagen Antileckagewände (16) auf dem Kantenabschnitt auf der Innenseite in die Breitenrichtung umfassen, und
die Kanten in die Längsrichtung der Aufrichtabschnitte, die sich in Richtung der Hautseite der Antileckagewände (16) erheben, auf der Außenweite in die Längsrichtung, bezogen auf die Flügel-Aktionslinien (34), angeordnet sind.

9. Saugfähiger Artikel nach einem der Ansprüche 1 bis 8, wobei:
die Flügelabschnitte (3) jeweils eine Verstärkungslage (9) umfassen, die die Steifigkeit der Flügelabschnitte (3) erhöht, und
die Kanten auf der Innenseite in der Breitenrichtung der Verstärkungslage (9) an der selben Position angeordnet sind oder auf den Außenseiten in der Breitenrichtung, bezogen auf die Linien die beide Kanten der Flügelabschnitte (3) in die Längsrichtung verbinden, angeordnet sind.

10. Saugfähiger Artikel nach einem der Ansprüche 1 bis 6, wobei:
der Körperabschnitt (2) ferner ein Paar Seitenlagen (8), vorgesehen auf beiden Seiten in der Breitenrichtung der Vorderlage (4), umfasst,
die Flügelabschnitte (3) jeweils die Rücklage (5) und die Seitenlage (8) umfassen, die sich in die Breitenrichtung von der Seitenkante an der Mitte der Längsrichtung des Körperabschnitts (2) erstrecken,
die Flügelabschnitte (3) jeweils einen durch Verkleben der Seitenlage (8) und der Rücklage (5) durch Heißprägen gebildeten Abdichtabschnitt (13) auf beiden Seiten in der Längsrichtung aufweisen, und
die Kanten auf den Innenseiten der Abdichtabschnitte (13) in die Breitenrichtung an der gleichen Position angeordnet sind oder an der Auffenseite in der Breitenrichtung, bezogen auf Linien, die beide Kanten der Flügelabschnitte (3) in die Längsrichtung verbinden, angeordnet sind.

11. Saugfähiger Artikel nach einem der Ansprüche 1 bis 8, wobei:
die Flügelabschnitte (3) jeweils eine Öffnung (35) umfassen, die durch die Dickenrichtung verläuft, und
die Kanten auf der Innenseite in die Breitenrichtung der Öffnungen (35) an der selben Position angeordnet sind oder auf den Außenseiten in der Breitenrichtung, bezogen auf Linien, die beide Kanten der Flügelabschnitte (3) in die Längsrichtung verbinden, angeordnet sind.

## Revendications

1. Article absorbant ayant une direction de la longueur et une direction de la largeur, et comprenant une section de corps (2) comprenant une feuille avant perméable aux liquides (4) fournie côté peau, une feuille arrière imperméable aux liquides (5) fournie côté vêtement, et une couche absorbante retenant les liquides (6) fournie entre la feuille avant (4) et la feuille arrière (5), et ayant une feuille de polymère super-absorbant retenant les liquides (7) fournie entre la feuille avant (4) et la couche absorbante (6) et comprenant un corps absorbant composé seulement d'un polymère super-absorbant, avec des feuilles perméables aux liquides (6b, 6c) prenant en sandwich le corps absorbant, et une paire de sections d'ailettes (3) s'étendant dans la direction de la largueur depuis les deux bords (21) au centre dans la direction de la longueur de la section de corps (2),
dans lequel la feuille avant (4) et la feuille de polymère super-absorbant (7) sont collées,
la paire de sections d'ailettes (3) ont chacune un bord (32) dans la direction de la longueur,
les positions des bords (32) dans la direction de la longueur des sections d'ailettes (3) chacune est le point où les bordures des sections d'ailettes (3) s'intersectent avec les bords latéraux (21) de la section de corps (2),
les lignes d'action des sections d'ailettes (34) sont définies le long de la direction de la largueur en reliant les bords (32) desdites sections d'ailettes (3),
des bords (73) dans la direction de la longueur de la feuille de polymère super-absorbant (7) sont positionnés sur le côté extérieur dans la direction de la longueur, par rapport aux lignes d'action des sections d'ailettes (34), et
les lignes d'action des sections d'ailettes (34) sont positionnées aux même positions ou sur les côtés extérieurs dans la direction de la longueur, par rapport à une position de pliage lorsque l'article absorbant (1, 1A) est plié en trois sections dans la direction de la longueur pour un emballage individuel de l'article absorbant (1, 1A),

2. Article absorbant selon la revendication 1, dans lequel :
la feuille de polymère super-absorbant (7) a deux ou plusieurs régions avec présence de polymère super-absorbant (71) où le corps absorbant est présent, et une région avec absence de polymère super-absorbant (72) où le corps absorbant est essentiellement absent,
les deux ou plusieurs régions avec présence de polymère super-absorbant (71) s'étendant dans la direction de la longueur et étant alignées dans la direction de la largeur, et
les lignes d'action des sections d'ailettes (34) étant positionnées sur les côtés extérieurs dans la direction de la longueur par rapport aux bords dans la direction de la longueur des deux ou plusieurs régions avec présence de polymère super-absorbant (71).

3. Article absorbant selon la revendication 1 ou 2, dans lequel :
une paire de rainures comprimées (14), formées par compression de la section de corps (2) dans la direction de l'épaisseur par gaufrage à chaud, s'étendant dans la direction de la longueur et alignées dans la direction de la largeur, et allant de la feuille avant (4) à l'intérieur de la couche absorbante, sont fournies côté peau de la section de corps (2),
l'espacement de la paire de rainures comprimées (14) dans la direction de la largeur augmentant du centre dans la direction de la longueur vers les côtés extérieurs dans la direction de la longueur.

4. Article absorbant selon la revendication 3, dans lequel les bords des rainures comprimées (14) dans la direction de la longueur sont positionnés sur les côtés intérieurs dans la direction de la longueur par rapport aux bords dans la direction de la longueur de la feuille de polymère super-absorbant (7).

5. Article absorbant selon la revendication 3 ou 4, dans lequel deux des régions avec présence de polymère super-absorbant (71) des deux ou plusieurs régions avec présence de polymère super-absorbant (71) sont fournies sur les côtés extérieurs respectifs de la paire de rainures comprimées (14) dans la direction de la largeur.

6. Article absorbant selon l'une quelconque des revendications 1 à 5, dans lequel les bords dans la direction de la longueur de la couche absorbante (6) sont aux même positions ou positionnés sur les côtés extérieurs dans la direction de la longueur, par rapport aux bords dans la direction de la longueur de la feuille de polymère super-absorbant (7).

7. Article absorbant selon l'une quelconque des revendications 1 à 6, dans lequel :
la section de corps (2) comprend en outre une paire de feuilles latérales (8) fournies des deux côtés dans la direction de la largeur de la feuille avant (4),
les feuilles latérales (8) ont des parois antifuite (16) sur les sections de bord sur le côté intérieur dans la direction de la largeur, et
les bords dans la direction de la longueur des sections dressées, qui se soulèvent vers le côté peau des parois antifuite (16), sont positionnés sur les côtés intérieurs dans la direction de la longueur, par rapport aux bords dans la direction de la longueur de la feuille de polymère super-absorbant (7).

8. Article absorbant selon l'une quelconque des revendications 1 à 6, dans lequel :
la section de corps (2) comprend en outre une paire de feuilles latérales (8) fournies des deux côtés dans la direction de la largeur de la feuille avant (4),
les feuilles latérales ont des parois antifuite (16) sur les sections de bord sur le côté intérieur dans la direction de la largeur, et
les bords dans la direction de la longueur des sections dressées, qui se soulèvent vers le côté peau des parois antifuite (16), sont positionnés sur les côtés extérieurs dans la direction de la longueur, par rapport aux lignes d'action des ailettes (34).

9. Article absorbant selon l'une quelconque des revendications 1 à 8, dans lequel :
les sections d'ailettes (3) ont chacune une feuille de renfort (9) qui augmente la rigidité de la section d'ailette (3), et
les bords sur les côtés intérieurs dans la direction de la largeur de la feuille de renfort (9) sont positionnés aux mêmes positions ou sont positionnés sur les côtés extérieurs dans la direction de la largeur, par rapport à des lignes reliant les deux bords des sections d'ailettes (3) dans la direction de la longueur.

10. Article absorbant selon l'une quelconque des revendications 1 à 6, dans lequel :
la section de corps (2) comprend en outre une paire de feuilles latérales (8) fournies des deux côtés dans la direction de la largeur de la feuille avant (4),
les sections d'ailettes (3) comprennent chacune la feuille arrière (5) et la feuille latérale (8), s'étendant dans la direction de la largeur du côté latéral au centre dans la direction de la longueur de la section de corps (2),
les sections d'ailettes (3) ont chacune une section de joint (13) formée par collage de la feuille latérale (8) et de la feuille arrière (5) par gaufrage à chaud, sur les deux côtés dans la direction de la longueur, et
les bords sur le côté intérieur de la section de joint (13) dans la direction de la largeur sont positionnés aux mêmes positions ou sont positionnés sur les côtés extérieurs dans la direction de la largeur, par rapport à des lignes reliant les deux bords des sections d'ailettes (3) dans la direction de la longueur.

11. Article absorbant selon l'une quelconque des revendications 1 à 8, dans lequel :
les sections d'ailettes (3) ont chacune une ouverture (35) traversant la direction de l'épaisseur, et les bords sur les côtés intérieurs dans la direction de la largeur des ouvertures (35) sont positionnés à la même position ou sont positionnés sur les côtés extérieurs dans la direction de la largeur, par rapport à des lignes reliant les deux bords des sections d'ailettes (3) dans la direction de la longueur.
